# EUROPÄISCHE PATENTSCHRIFT

(11) **EP 0 715 831 B1**
(45) Veröffentlichungstag und Bekanntmachung des Hinweises auf die Patenterteilung: **17.02.1999**
(21) Anmeldenummer: 95203283.7
(22) Anmeldetag: 29.11.1995
(51) Int. Cl.: A61B 17/22

(54) **Lithotripsiekombination mit einer Therapieeinheit**
Lithotripsy combination with a therapy unit
Ensemble de lithotripsie avec une unité de thérapie

(30) Priorität: 07.12.1994 DE 4443495
(43) Veröffentlichungstag der Anmeldung: 12.06.1996
(73) Patentinhaber: Philips Patentverwaltung GmbH, 22335 Hamburg (DE); Koninklijke Philips Electronics N.V., 5621 BA Eindhoven (NL)
(72) Erfinder: Schwieker, Horst, c/o Philips, D-22335 Hamburg (DE)
(74) Vertreter: Hartmann, Heinrich, Dipl.-Ing.

(56) Entgegenhaltungen:
- EP-A- 0 405 000
- EP-A- 0 538 659
- DE-A- 4 300 740
- US-A- 5 065 741
- US-A- 5 285 772

## Beschreibung

Die Erfindung betrifft eine Lithotripsiekombination mit einer Therapieeinheit und einer separaten, einen ersten Wagen umfassenden und mit der Therapieeinheit über eine Koppeleinheit koppelbaren Röntgen-Ortungseinheit, die einen Röntgenstrahler und einen Röntgen-Bildwandler zur Ortung in wenigstens zwei definierten Ortungsstellungen umfaßt. Eine solche Lithotripsiekombination ist aus der DE-PS 43 00 740 bekannt.

Lithotripter sind teuer, weil sie in der Lage sein müssen, in einem auf einem Lagerungstisch befindlichen Patienten ein Konkrement zu orten - z.B. mittels einer Röntgen-Ortungseinheit - und das geortete Konkrement dann zu zerstören. Der eingangs genannten bekannten Vorrichtung liegt daher die Überlegung zugrunde, die Wirtschaftlichkeit eines solchen Lithotripters dadurch zu verbessern, daß man zumindest einzelne Komponenten auch außerhalb der Lithotripsie einsetzt.

Zu diesem Zweck besteht die bekannte Lithotripsiekombination aus einer Therapieeinheit und einer separaten Röntgen-Ortungseinheit, wobei als Röntgen-Ortungseinheit ein handelsüblicher C-Bogen dient, der einen Röntgenstrahler und einen Röntgen-Bildwandler trägt und um eine durch die C-Bogenebene verlaufende horizontale Achse schwenkbar ist. Dieser C-Bogen ist auf einem Wagen angeordnet und kann einerseits in Verbindung mit der Therapieeinheit zur Ortung eines Konkrements benutzt werden und andererseits - unabhängig von der Therapieeinheit zu einer normalen Röntgenuntersuchung.

Bei der bekannten Vorrichtung besteht die Therapieeinheit aus einem Patientenlagerungstisch, in den - um eine horizontale Querachse schwenkbar - ein elektroakustischer Wandler integriert ist, der aus einer Vielzahl von kalottenförmig angeordneten piezoelektrischen Elementen besteht. Dieser Wandler erzeugt Ultraschallwellen, deren Intensität in einem durch die erwähnte Schwenkachse verlaufenden Fokus so groß werden kann, daß ein dort befindliches Konkrement zerstört wird. Zur Durchführung einer Lithotripsie wird der Wagen mit der Röntgen-Ortungseinheit an die Therapieeinheit herangefahren, und der Wandler wird durch die Koppelvorrichtung derart mit dem C-Bogen der Röntgen-Ortungseinheit gekoppelt, daß der C-Bogen den Schwenkbewegungen des Wandlers folgen kann.

Bei der bekannten Lithotripsiekombination kann nur eine Grobausrichtung zwischen Röntgen-Ortungseinrichtung und Therapiegerät erfolgen. Für eine genaue Ausrichtung wird im Fokus des Wandlers eine im Röntgenbild erscheinende Markierung, beispielsweise eine Bleikugel, angebracht, und der C-Bogen in die beiden Ortungsstellungen geschwenkt. Eine elektronische Zielmarke wird in den Röntgenbildern, die in diesen Ortungsstellungen von der Bleikugel aufgenommen werden, nachgeführt, und diese Nachführung der elektronischen Zielmarke wird gespeichert. Nachdem die Bleikugel entfernt und ein Patient auf dem Patientenlagerungstisch plaziert worden ist, wird die gespeicherte Einstellung bei der Darstellung der elektronischen Zielmarke auf einem das Röntgenbild zeigenden Monitor berücksichtigt.

Weiterhin ist aus der US-A-5285772 bereits eine Lithotripsiekombination mit einer auf einem ersten Wagen befindlichen Röntgen-Ortungseinheit und einer auf einem zweiten Wagen befindlichen Therapieeinheit bekannt. Zur Ausrichtung von Therapieeinheit und Röntgen-Ortungseinheit in einer einzigen Ortungsstellung sind auf der akustischen Achse des Stoßwellenerzeugers der Therapieeinheit zwei Bleimarken vorgesehen, von denen eine entfallen kann, wenn es möglich ist, den Zentralstrahl der Röntgen-Ortungseinheit und die akustische Achse des Stoßwellenerzeugers in einer definierten Winkelstellung zu arretieren.

Aufgabe der vorliegenden Erfindung ist es ebenfalls, eine Lithotripsiekombination vielseitiger verwendbar zu machen; jedoch sollen Röntgen-Ortungseinheit und Therapieeinheit wesentlich leichter aufeinander ausgerichtet werden können. Diese Aufgabe wird erfindungsgemäß dadurch gelöst, daß die Therapieeinheit aus einem zweiten Wagen und einem darauf befestigten Stoßwellenerzeuger mit einem bezüglich des Wagens ortsfesten Fokus besteht, daß die beiden Wagen oder damit fest verbundene Teile über die Koppeleinheit so miteinander koppelbar sind, daß die gekoppelten Wagen gemeinsam verfahrbar sind und daß die gekoppelten Wagen (103, 203) relativ zueinander in einer genau definierten Position derart angeordnet sind, daß der Fokus des Stoßwellenerzeugers zumindestens in den Ortungsstelllungen immer auf dem Zentralstrahl liegt, der den Röntgenstrahler und den Röntgen-Bildwandler miteinander verbindet.

Bei der Erfindung besteht die Therapieeinheit also lediglich aus einem zweiten Wagen und einem darauf befestigten Stoßwellenerzeuger. Durch die Koppeleinheit werden nur die beiden Wagen oder damit fest verbundene Teile miteinander gekoppelt, so daß der Röntgenstrahler und der Röntgen-Bildwandler auch bei miteinander gekoppelten Wagen (gemeinsam) und unabhängig von dem Stoßwellenerzeuger in die Ortungsstellungen bewegbar sind. Die Therapieeinheit und die Röntgen-Ortungseinheit sind gemeinsam verfahrbar. Sie sind durch die Koppelvorrichtung relativ zueinander in einer genau definierten Position, in der der Fokus des Stoßwellenerzeugers auf dem Zentralstrahl liegt, der jeweils den Röntgenstrahler und den Röntgen-Bildwandler miteinander verbindet. Ein zusätzliches Ausrichtverfahren wie bei der bekannten Lithotripsiekombination ist deshalb nicht erforderlich.

Während bei der bekannten Lithotripsiekombination die Ortungseinheit im Prinzip eine beliebige handelsübliche Ortungseinheit vom C-Bogentyp sein kann, müssen bei der Erfindung die Röntgen-Ortungseinheit und die Therapieeinheit konstruktiv aneinander angepaßt sein, so daß sich im gekoppelten Zustand der Fokus immer im Zentralstrahl der Ortungsstellungen befindet. Gleichwohl kann die Röntgen-Ortungseinheit nach dem Abkoppeln der Therapieeinheit auch für andere Röntgenuntersuchungen eingesetzt werden.

Es sei an dieser Stelle erwähnt, daß aus der US-PS 5,065,741, insbesondere Fig. 6 bereits eine Lithotripsieanlage bekannt ist, die aus drei separaten Komponenten besteht: Einem Patientenlagerungstisch, einer Röntgen-Ortungseinheit mit einem C-Bogen und einer verfahrbaren Therapieeinheit, die - ebenfalls an einem C-Bogen - einen Stoßwellenerzeuger enthält. Die Anlage kann ohne die Therapieeinheit für konventionelle Röntgenuntersuchungen benutzt werden. Für Lithotripsiezwecke wird die Lithotripsieeinheit so an dem Patientenlagerungstisch plaziert, daß sich Röntgen-Ortungseinheit und Lithotripsieeinheit beiderseits des Tisches befinden. Es ist allerdings keine Möglichkeit vorgesehen, die Therapieeinheit und die Röntgen-Ortungseinheit aufeinander auszurichten.

Eine bevorzugte Weiterbildung der Erfindung sieht vor, daß auf dem ersten Wagen ein C-Bogen um wenigstens eine horizontale Achse schwenkbar gelagert ist, der den Röntgenstrahler und den Röntgen-Bildwandler trägt. Eine C-Bogen-einheit ist auch für konventionelle Röntgenuntersuchungen vielseitig verwendbar.

Für die Röntgenortung mit einer solchen C-Bogen-Einheit gibt es verschiedene Möglichkeiten. Nach einer ersten Ausgestaltung wird die Tatsache ausgenutzt, daß der C-Bogen um eine horizontale, zu der durch den C-Bogen definierten Ebene parallele Achse in die verschiedenen Ortungsstellungen schwenkbar ist, die im gekoppelten Zustand der beiden Wagen durch den Fokus des Stoßwellenerzeugers verläuft. Die Lithotripsiekombination wird dabei so an einen Untersuchungstisch herangefahren, auf dem der Patient gelagert ist, daß die Schwenkachse etwa senkrecht zur Tischlängsrichtung verläuft; diese Schwenkmöglichkeit wird auch bei der bekannten Lithotripsiekombination ausgenutzt.

Bei einer zweiten Ortungsmöglichkeit wird in einer anderen Ausgestaltung der Erfindung die Tatsache ausgenutzt, daß der C-Bogen um eine horizontale, zu der durch den C-Bogen definierten Ebene senkrechte Achse in wenigstens zwei Ortungsstellungen schwenkbar ist, und daß im gekoppelten Zustand der beiden Wagen der Schnittpunkt der Zentralstrahlen in den beiden Ortungsstellungen mit dem Fokus des Stoßwellenerzeugers zusammenfällt. In diesem Fall wird der C-Bogen also um eine zweite Schwenkachse geschwenkt, die etwa parallel zur Längsrichtung des Patientenlagerungstisches verläuft und auf der sich der Krümmungsmittelpunkt des C-Bogens befindet. Diese Ortungsmöglichkeit kann bei der bekannten Einrichtung nicht ausgenutzt werden, weil deren C-Bogen im gekoppelten Zustand nicht mehr um diese Achse geschwenkt werden kann. Bei der Erfindung ist dies hingegen ohne weiteres möglich, weil die Koppelvorrichtung an dem ersten Wagen oder einem damit starr verbundenen Teil angreift, aber nicht an dem C-Bogen. Dieser ist daher unabhängig von der Therapieeinheit, insbesondere dem Stoßwellenerzeuger um diese zweite Achse schwenkbar.

Für eine Lithotripsie-Behandlung wird die Lithotripsiekombination an einen Patientenlagerungstisch herangefahren, auf dem der zu behandelnde Patient gelagert ist. Eine bevorzugte Weiterbildung der Erfindung sieht vor, daß der Patientenlagerungstisch mit einer vorzugsweise auswechselbaren Tischplatte versehen ist, die an einer Längsseite einen Ausschnitt für die Lithotripsiekombination aufweist. Der Stoßwellenerzeuger auf dem zweiten Wagen befindet sich nämlich - zumindest größtenteils - unterhalb des Patientenlagerungstisches, so daß der Stoßwellenerzeuger durch den Ausschnitt hindurch auf den Patienten einwirken kann (wobei der Zwischenraum zwischen dem Stoßwellenerzeuger und dem Patienten in bekannter Weise durch ein Wasserkissen ausgefüllt ist). Nach dem Entkoppeln der Lithotripsiekombination und dem Austausch der mit dem Ausschnitt versehenen Tischplatte gegen eine Tischplatte ohne Ausschnitt kann der Patientenlagerungstisch und die Röntgen-Ortungseinheit für konventionelle Röntgenuntersuchungen benutzt werden. Dies ist mit der eingangs erwähnten bekannten Anordnung nicht möglich, weil der unterhalb der Tischplatte befindliche elektroakustische Wandler zur Zerstörung von Konkrementen eine solche Untersuchung behindern würde.

Die Erfindung wird nachstehend anhand der Zeichnung näher erläutert. Es zeigen
- Fig. 1: die Lithotripsiekombination in entkoppeltem Zustand in einer Seitenansicht,
- Fig. 2: Teile der in Fig. 1 dargestellten Kombination in perspektivischer Darstellung,
- Fig. 3: die gekoppelte Lithotripsiekombination in Verbindung mit einem Patientenlagerungstisch,
- Fig. 4: eine Draufsicht auf den Patientenlagerungstisch und
- Fig. 5: eine Frontansicht der in Fig. 3 dargestellten Kombination.

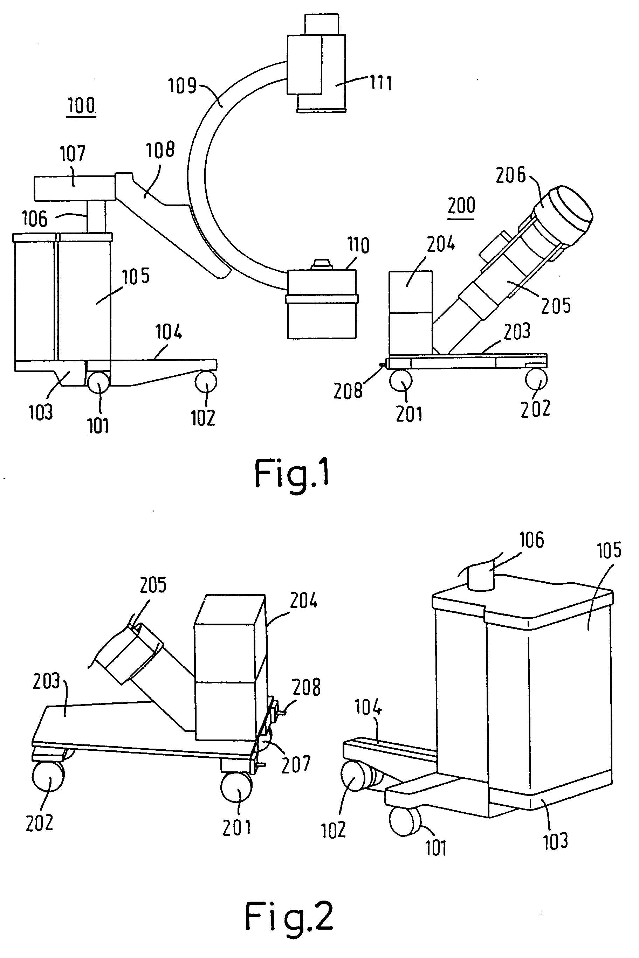

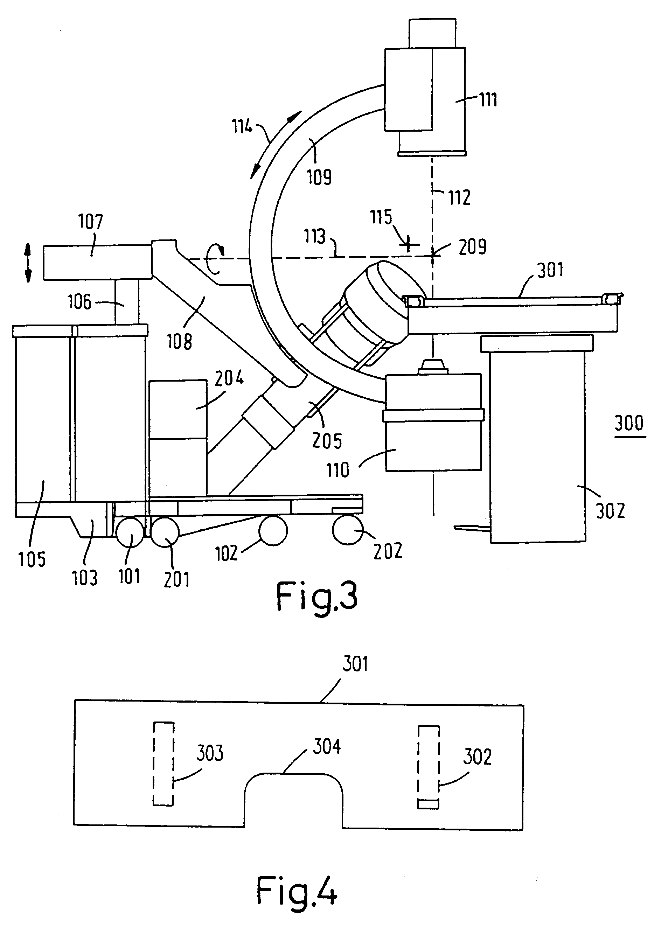

In Fig. 1 ist die Röntgenortungseinheit mit 100 und die Therapieeinheit mit 200 bezeichnet. Die Röntgenortungseinheit umfaßt einen auf Rollen 101 und 102 verfahrbaren Wagen 103, der einen Mittelteil 104 aufweist, an dem die vorderen Rollen 102 befestigt sind. In dem auf dem Wagen befindlichen Gehäuse 105 sind einerseits die elektrischen Komponenten zum Betrieb der Röntgenortungseinheit enthalten und ist andererseits eine vertikale Hubsäule 106 in ihrer Längsrichtung verschiebbar angeordnet. Die Hubsäule trägt an ihrem oberen Ende einen horizontalen Tragarm 107, in dem ein Führungssegment 108 um eine horizontale Achse schwenkbar gelagert ist. In dem Führungssegment 108 ist ein C-Bogen 109 gelagert, der an seinem einen Ende einen Röntgenstrahler 110 und an seinem anderen Ende einen Röntgen-Bildwandler 111, z.B. einen Röntgenbildverstärker trägt. Der C-Bogen 109 kann in dem Führungssegment um eine durch seinen Krümmungsmittelpunkt (115; Fig. 3) senkrecht zur Zeichenebene der Fig. 1 verlaufende Achse geschwenkt werden. Außerdem ist eine Schwenkung des C-Bogens mitsamt dem Führungssegment 108 um eine horizontale Achse möglich, die in der Zeichenebene bzw. in der durch den C-Bogen 109 gebildeten Ebene liegt und die in Fig. 3 mit 113 bezeichnet ist.

Die Therapieeinheit 200 umfaßt ebenfalls einen auf Rollen 201 und 202 verfahrbaren Wagen 203, der den Stoßwellenerzeuger trägt. Der Stoßwellenerzeuger umfaßt einen in einem Gehäuse 204 untergebrachten elektrischen Generator und einen an einem winkelförmigen Träger 205 befestigten Stoßwellenkopf 206. Der Stoßwellenkopf 206 kann in bekannter Weise ein Rotationsellipsoid enthalten, in dem mittels einer Funkenstrecke eine von dem erwähnten Generator gespeiste elektrische Entladung stattfindet, durch die Stoßwellen erzeugt werden, deren Energie auf einen Punkt - den sogenannten Fokus - konzentriert wird. Dieser Fokus liegt oberhalb des Stoßwellenkopfes 206 in einer genau definierten Position bezüglich des Wagens 203.

Wie aus Fig. 2 ersichtlich, befindet sich unterhalb des Wagens 203 ein Tunnel 207, der der Aufnahme des Mittelteils 104 der Röntgenortungseinheit 100 dient. Wenn der Wagen 103 der Röntgenortungseinheit auf den Wagen 203 des Stoßwellenerzeugers zu geschoben wird und der Mittelteil 104 in den Tunnel eindringt, werden die beiden Wagen in Längsrichtung aufeinander ausgerichtet, wobei am Ende der Verschiebung Koppelstifte 208 am Wagen 203 in dazu komplementär geformten, nicht näher dargestellten Koppelöffnungen am Wagen 103 einrasten. Beim Einfahren des Mittelteils 104 wird gleichzeitig der Wagen 203 geringfügig angehoben, so daß seine Rollen 201 und 202 über dem Boden schweben. Die durch die Kopplung gebildete Lithotripsiekombination ist dann auf den Rollen 101 und 102 des Wagens 103 der Röntgen-Ortungseinheit verfahrbar. Durch eine zusätzliche, nicht näher dargestellte Verriegelungsvorrichtung kann dabei sichergestellt werden, daß es bei dieser gekoppelten Stellung bleibt.

Die Kopplungseinheit, über die die beiden Wagen 103, 203 miteinander koppelbar sind, kann auch auf andere Weise aufgebaut sein. Wesentlich ist dabei lediglich, daß die beiden Wagen und/oder die damit fest verbundenen Teile so gestaltet sind, daß sie nur in einer genau definierten Position relativ zueinander arretiert bzw. miteinander gekoppelt und gemeinsam verfahren werden können.

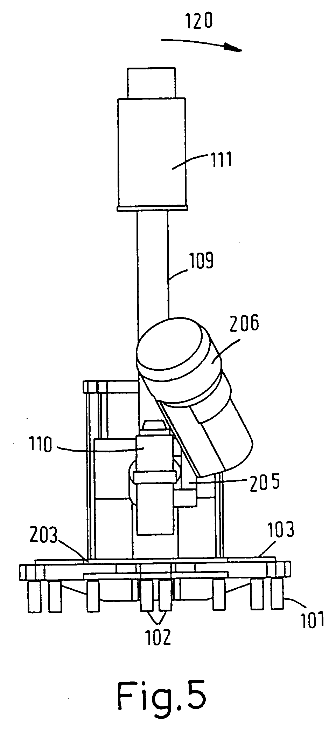

Fig. 3 zeigt die gekoppelte Lithotripsiekombination in Verbindung mit einem Patientenlagerungstisch 300. Wie insbesondere aus Fig. 5 ersichtlich, befindet sich bei vertikaler Stellung des C-Bogens 109 der Stoßwellenkopf 206 und der Träger 205 seitlich vom C-Bogen. Jedoch ist der Stoßwellenkopf aus dem Träger 205 so angeordnet, daß sein Fokus 209 in der Ebene des C-Bogens liegt und zwar auf dem sogenannten Zentralstrahl 112, der einer Verbindungsgeraden zwischen dem Mittelpunkt des Eingangsleuchtschirms des Röntgenbildverstärkers 111 und dem die Röntgenstrahlung im Röntgenstrahler 110 emittierenden Brennfleck entspricht. Der Stoßwellenkopf 206 kann gegebenenfalls noch von dem Röntgenstrahlenbündel erfaßt und auf dem Röntgen-Bildwandler 111 abgebildet werden; jedoch geht zumindestens der Zentralstrahl 112 an dem Stoßwellenkopf vorbei.

Der Patientenlagerungstisch 300 umfaßt eine Tischplatte 301 und Lagerböcke 302 und 303, die die vertikale Verschiebung der Tischplatte 301 gestatten. Darüberhinaus ist die Tischplatte als sogenannte schwimmende Tischplatte ausgebildet, d.h. sie kann in ihrer Ebene verschoben werden. Für den Stoßwellenkopf hat die Tischplatte eine Ausnehmung 304, wie in Fig. 4 dargestellt. Die im Stoßwellenkopf erzeugten Stoßwellen können daher durch diesen Ausschnitt hindurch über ein aufpumpbares Wasserkissen auf einen auf der Tischplatte 301 befindlichen Patienten einwirken, ohne mit der Tischplatte in Wechselwirkung zu treten.

Eine erste Ortungsmöglichkeit besteht darin, den C-Bogen 109 mitsamt dem Führungssegment 108 um die in der C-Bogen-Ebene liegende horizontale Achse 113 in eine zweite Ortungsstellung zu schwenken, und zwar in Richtung des Pfeils 120 (vergl. Fig. 5) um einen Winkel von etwa 30°, wobei sich keine Kollision mit dem Meßkopf 206 bzw. dem Träger 205 ergibt. Auch in dieser zweiten Schwenkstellung muß der Fokus 209 auf dem Zentralstrahl 112 liegen. Dies wird dann erreicht, wenn der Träger 107 auf der Hubsäule 106 in eine solche Stellung gebracht wird, daß die Schwenkachse 113 die gleiche Höhe hat wie der Fokus 209. Der Schnittpunkt der Zentralstrahlen 112 in den beiden Ortungsstellungen fällt dann mit dem Fokus 209 zusammen und liegt außerdem auf der Schwenkachse 113. Um die erforderliche Höhe der Schwenkachse 113 sicherzustellen, sollte die Hubsäule 106 an der betreffenden Höheneinstellung Markierungen aufweisen oder eine Raststellung, so daß die erwünschte Stellung reproduzierbar erreicht werden kann.

Eine bevorzugte weitere Möglichkeit zur Ortung besteht darin, bei senkrechter Stellung des C-Bogens den C-Bogen 109 innerhalb des Führungssegments 108 um eine durch seinen Krümmungsmittelpunkt 115 senkrecht zur Zeichenebene der Fig. 3 verlaufende Achse zu schwenken, wie durch den Pfeil 114 angedeutet. Wenn der Krümmungsmittelpunkt 115 auf dem Zentralstrahl 112 und auf der horizontalen Achse 113 liegt, ist eine Ortung mit einer solchen Schwenkung - und zwar in beliebigen Ortungsstellungen ohne weiteres möglich.

In vielen Fällen verläuft die horizontale Schwenkachse 113 jedoch nicht durch den Krümmungsmittelpunkt, sondern durch den Schwerpunkt der aus den Komponenten 108, 109, 110 und 111 bestehenden C-Bogeneinheit, damit eine manuelle Schwenkung um die Achse 113 möglich wird, und außerdem verläuft auch der Zentralstrahl nicht durch den Krümmungsmittelpunkt 112. Eine solche Situation ist in Fig. 3 dargestellt, wo der Krümmungsmittelpunkt 115 außerhalb des Zentralstrahls 112 und außerhalb der Schwenkachse 113 liegt. Auch in diesem Fall schneiden sich aber in zwei verschiedenen Ortungsstellungen die Zentralstrahlen 112 in einem Punkt. Dieser Schnittpunkt fällt mit dem Fokus 209 zusammen, wenn die Hubsäule 106 auf eine bestimmte Höhe eingestellt wird, die in der Regel nicht mit derjenigen Höhe zusammenfällt, in der die Schwenkachse 113 den Fokus 209 schneidet. Deshalb muß an der Hubsäule 106 wenigstens noch eine weitere Markierung bzw. Raststellung vorgesehen sein, damit auch eine Ortung durch Schwenkung des C-Bogens um seinen Krümmungsmittelpunkt 115 möglich wird.

Man kann mit dieser Lithotripsie-Anlage auch konventionelle Röntgenuntersuchungen durchführen. Dazu muß lediglich die Therapieeinheit 200 abgekoppelt und beiseite gefahren und ggf. die Tischplatte 301 gegen eine solche ohne Ausschnitt 304 ausgetauscht werden. Da man somit mit den gleichen Komponenten einerseits Lithotripsie-Behandlungen und andererseits Röntgenuntersuchungen durchführen kann, ergibt sich eine erhöhte Wirtschaftlichkeit.

## Patentansprüche

1. Lithotripsiekombination mit einer Therapieeinheit (200) und einer separaten, einen ersten Wagen (103) umfassenden und mit der Therapieeinheit über eine Koppeleinheit (208) koppelbaren Röntgen-Ortungseinheit (100), die einen Röntgenstrahler (111) und einen Röntgen-Bildwandler (110) zur Ortung in wenigstens zwei definierten Ortungsstellungen umfaßt,
dadurch gekennzeichnet, daß die Therapieeinheit (200) aus einem zweiten Wagen (203) und einem darauf befestigten Stoßwellenerzeuger (204, 206) mit einem bezüglich des Wagens (203) ortsfesten Fokus (209) besteht, daß die beiden Wagen (103, 203) oder damit fest verbundene Teile über die Koppeleinheit (208) so miteinander koppelbar sind, daß die gekoppelten Wagen gemeinsam verfahrbar sind und daß die gekoppelten Wagen (103, 203) relativ zueinander in einer genau definierten Position derart angeordnet sind, daß der Fokus (209) des Stoßwellenerzeugers zumindestens in den Ortungsstellungen immer auf dem Zentralstrahl (112) liegt, der den Röntgenstrahler (110) und den Röntgen-Bildwandler (111) miteinander verbindet.

2. Lithotripsiekombination nach Anspruch 1
dadurch gekennzeichnet, daß auf dem ersten Wagen (103) ein C-Bogen (109) um wenigstens eine horizontale Achse (113, 115 schwenkbar gelagert ist, der den Röntgenstrahler (110) und den Röntgen-Bildwandler (111) trägt.

3. Lithotripsiekombination nach Anspruch 2,
dadurch gekennzeichnet, daß der C-Bogen (119) um eine horizontale, zu der durch den C-Bogen definierten Ebene parallele Achse (113) in die verschiedenen Ortungsstellungen schwenkbar ist, die im gekoppelten Zustand der beiden Wagen durch den Fokus (209) des Stoßwellenerzeugers (204, 206) verläuft.

4. Lithotripsiekombination nach Anspruch 2,
dadurch gekennzeichnet, daß der C-Bogen um eine horizontale, zu der durch den C-Bogen definierten Ebene senkrechte Achse (115) in wenigstens zwei Ortungsstellungen schwenkbar ist, und daß im gekoppelten Zustand der beiden Wagen der Schnittpunkt der Zentralstrahlen )112)in den beiden Ortungsstellungen mit dem Fokus (209) des Stoßwellenerzeugers zusammenfällt.

5. Lithotripsie-Anlage mit einem Patientenlagerungstisch (300) und einer Lithotripsiekombination nach Anspruch 1,
dadurch gekennzeichnet, daß der Patientenlagerungstisch mit einer vorzugsweise auswechselbaren Tischplatte (301) versehen ist, die an einer Längsseite einen Ausschnitt (304) für die Lithotripsiekombination (100, 200) aufweist.

## Claims

1. A lithotripsy combination, comprising a therapy unit (200) and a separate X-ray locating unit (100) which comprises a first carriage (103) and can be coupled to the therapy unit via a coupling unit (208), said X-ray locating unit comprising an X-ray source (111) and an X-ray image converter (110) for locating in at least two defined locating positions,
characterized in that the therapy unit (200) consists of a second carriage (203) and a shockwave generator (204, 206) which is mounted thereon and has a focus (209) which is stationary relative to the carriage (203), that the two carriages (103, 203), or parts rigidly connected thereto, can be coupled to one another via the coupling unit (208) in such a manner that the coupled carriages can be displaced together and that the coupled carriages (103, 203) are arranged in an accurately defined position relative to one another in such a manner that, at least in the locating positions, the focus (209) of the shockwave generator is always situated on the central line (112) which interconnects the X-ray source (110) and the X-ray image converter (111).

2. A lithotripsy combination as claimed in Claim 1,
characterized in that on the first carriage (103) a C-arm (109) is journalled so as to be pivotable about at least one horizontal axis (113, 115), which C-arm, supports the X-ray source (110) and the X-ray image converter (111).

3. A lithotripsy combination as claimed in Claim 2,
characterized in that the C-arm (119) is pivotable to the various locating positions about a horizontal axis (113) which extends parallel to the plane defined by the C-arm, said axis extending through the focus (209) of the shockwave generator (204, 206) in the coupled condition of the two carriages.

4. A lithotripsy combination as claimed in Claim 2,
characterized in that the C-arm is pivotable to at least two locating positions about a horizontal axis (115) which extends perpendicularly to the plane defined by the C-arm, and that in the coupled condition of the two carriages the point of intersection of the central lines (112) coincides with the focus (209) of the shockwave generator in the two locating positions.

5. A lithotripsy system comprising a patient table (300) and a lithotripsy combination as claimed in Claim 1,
characterized in that the patient table comprises a preferably exchangeable table top (301) which is provided with a cut-out (304) for the lithotripsy combination (100, 200) at a longitudinal side.

## Revendications

1. Combinaison de lithotripsie avec une unité thérapeutique (200) et une unité de localisation radiographique (100) séparée comprenant un premier chariot (103) et susceptible d'être couplée avec l'unité thérapeutique par l'intermédiaire d'une unité d'accouplement (208), laquelle présente un générateur de rayons X (111) et un transformateur d'images radiographiques (110) en vue de la localisation dans au moins deux positions de localisation définies,
caractérisée en ce que l'unité thérapeutique (200) se compose d'un deuxième chariot (203) et d'un générateur d'ondes de choc (204, 206) fixé à celui-ci avec un foyer (209) stationnaire par rapport au chariot (203), que les deux chariots (103, 203) ou les pièces fixées rigidement à celui-ci peuvent être couplés entre eux par l'intermédiaire de l'unité d'accouplement (208) de telle sorte que les chariots couplés soient déplaçables ensemble et que les chariots couplés (103, 203) sont disposés dans une position relative définie avec précision de telle sorte que le foyer (209) du générateur d'ondes de choc se trouve toujours, du moins dans les positions de localisation, sur le faisceau central (112) qui relie le générateur de rayons X (110) et le transformateur d'images radiographiques (111).

2. Combinaison de lithotripsie selon la revendication 1,
caractérisée en ce qu'un arc en C (109) est logé sur le premier chariot (103) à pivotement autour d'au moins un axe horizontal (113, 115) qui porte le générateur de rayons X (110) et le transformateur d'images radiographiques (111).

3. Combinaison de lithotripsie selon la revendication 2,
caractérisée en ce que l'arc en C (119) peut pivoter dans les différentes positions de localisation autour d'un axe (113) horizontal parallèle au plan défini par l'arc en C qui s'étend lorsque les deux chariots sont couplés par le foyer (209) du générateur d'ondes de choc (204, 206).

4. Combinaison de lithotripsie selon la revendication 2,
caractérisée en ce que l'arc en C peut pivoter autour d'un axe (115) horizontal perpendiculaire au plan défini par l'arc en C dans au moins deux positions de localisation et, lorsque les deux chariots sont couplés, le point d'intersection des faisceaux centraux (112) coïncide dans les deux positions de localisation avec le foyer (209) du générateur d'ondes radio.

5. Installation de lithotripsie avec une table d'examen du patient (300) et une combinaison de lithotripsie selon la revendication 1,
caractérisée en ce que la table d'examen du patient est dotée d'un dessous de table (301), de préférence remplaçable, qui présente sur une face longitudinale une découpe (304) pour la combinaison de lithotripsie (100, 200).
